# EUROPEAN PATENT SPECIFICATION

(11) **EP 1 025 235 B1**
(45) Date of publication and mention of the grant of the patent: **31.05.2006**
(21) Application number: 98946599.2
(22) Date of filing: 09.10.1998
(51) Int. Cl.: C12N 15/53, C12N 9/02, C12P 21/02

(54) **EXPRESSION SYSTEM**
EXPRESSIONSSYSTEM
SYSTEME D'EXPRESSION

(30) Priority: 25.10.1997 GB 9722481
(43) Date of publication of application: 09.08.2000
(73) Proprietor: THE SECRETARY OF STATE FOR DEFENCE, Farnborough, Hampshire GU14 OLX (GB)
(72) Inventor: SQUIRRELL, David, James, Salisbury, Wiltshire SP4 0JQ (GB); PRICE, Rachel, Louise, Salisbury, Wiltshire SP4 0JQ (GB); MURPHY, Melanie, Jane, Salisbury, Wiltshire SP4 0JQ (GB)
(74) Representative: Beckham, Robert William
(86) International application number: PCT/GB1998/003034
(87) International publication number: WO 1999/022004

(56) References cited:
- WO-A-94/17202
- WO-A-96/22376
- US-A- 5 030 563
- US-A- 5 538 862
- BELINGA, HONORE FAUSTIN ET AL: "Firefly luciferase purification using polyethylene glycol and Dyematrex Orange A" J. CHROMATOGR., A (1995), 695(1), 33-40 CODEN: JCRAEY, XP004022931
- MONNOT M. ET AL: 'Circular dichroism investigation of Escherichia coli adenylate kinase' J.BIOL.CHEM. vol. 262, no. 6, 25 February 1987, pages 2502 - 2506
- HOWELL M.J.; HARGREAVES J.J.: 'Cloning and expression of Taenia ovis antigens in Escherichia coli' MOL.BIOCHEM.PARASITOL. vol. 28, 1988, pages 21 - 30
- DOHMEN ET AL SCIENCE vol. 263, 04 March 1994, pages 1273 - 1276, XP002142706
- GRALLERT ET AL CURRENT GENETICS vol. 32, 1997, pages 27 - 31, XP008024858
- OHYA ET AL GENETICS vol. 138, 1994, pages 1041 - 1054, XP000915010
- CHAKSHUSHMATHI ET AL PROC.NATL.ACAD.SCI.USA vol. 101, no. 21, 25 May 2004, pages 7925 - 7930
- "Temperature-sensitive mutations affecting kinetic steps in protein-folding pathways", King J. et al., p.109-121, in "PROTEIN ENGINEERING", Eds Oxender D.L. & Fox C.F.; Alan R. Liss, Inc, New York 1987

## Description

The present invention relates to a method of obtaining polypeptides or protein products such as enzymes and in particular luciferases, which are substantially free of undesired contaminants such as adenylate kinase, usually found in expression products, to recombinant host cells, vectors and nucleotide sequences useful in these methods.

Recombinant DNA technology has allowed the production of a vast range of useful protein and polypeptide products efficiently. Essentially, host cells which may be eukaryotic or prokaryotic, are engineered so that they express the desired products. Culture of the recombinant cells results in the production of the desired products, sometimes in large quantities. Particularly useful host cells are prokaryotic cells such as bacteria like *E. coli* or *Bacillus spp..* These cells multiply rapidly and so culture of the cells means that large quantities of the desired products may be obtained either by lysis of the cells, or by extraction from culture supernatant if the cells can be induced to secrete these products. The desired product must then be extracted from the culture medium and purified so that it is free of all the other proteins present as a result of the cell culture process.

Purification can sometimes be difficult to achieve as the culture medium contains a large amount of other proteins and products, some of which may have similar properties such as size, charge or affinity for a particular substrate to the desired protein, making complete purification using for example chromatographic techniques difficult. For many applications, small quantities of contaminant proteins may not present a significant problem in terms of the end use of the product. For other applications however, the presence of even minute quantities of particular undesired products may be extremely damaging with regard to the utility of the product. A particular case may relate to enzymes found in host cells. If the activity of such molecules impedes or is contrary to that of the desired product, even tiny amounts present as a contaminant may hinder or obstruct the end use of the product.

Where the damaging contaminant is the product of a non-essential gene of the host cell, the problem may be addressed by inactivating that gene, for example by deletion, mutation or even by using anti-sense RNA constructs to "switch off" the gene. However, where the gene product is essential for the survival of the host cell such an approach is not possible as clearly, the host cell would not survive and so the production process would cease. The same may apply where inactivation reduces the efficiency of the cell by a significant amount so that the production process is rendered non-viable.

For example, luciferase enzyme is well known as a labelling tool in biological research. It is a useful enzyme in that when combined with luciferin and ATP, it provides a useful signalling system, providing a fluorescent signal which can be read easily using for example luminometer devices. Luciferase can be isolated from natural sources such as fireflies and some beetles. For large scale production however, luciferase is generally produced by expression from a prokaryotic host, such as *E. coli.*

One particular application to which luciferase may be put is in an assay for detection of cellular components such as ATP or enzymes such as adenylate kinase (AK), as described in European Patent Application No. 94904295.6. Such assays are useful in detecting the presence of microorganisms in a particular enviroment. For these purposes, the presence of cellular components which are the target of the assay in the luciferase reagent will produce levels of "background" noise which will have to be taken account of when interpreting results obtained using these products. This is a particular problem in the adenylate kinase assay, which has a high level of sensitivity.

Adenylate kinase is the enzyme which catalyses the reaction which converts ADP to ATP, the essential energy component of the cell. In the assay mentioned above, the amount of AK is measured by adding ADP to the reagent mixture which is converted to ATP by any AK present. The ATP product is then detected using luciferase/luciferin system. The use of AK as the substrate in the assay produces an amplication of the signal many times over. This is very useful when very small quantities of cellular content, for instance, very small numbers of microrganisms are present in the sample.

However, equally, any residual AK in the luciferase reagent used in the assay will generate a similarly amplified signal and may generate false positive results. For this reason, highly stringent purification of the luciferase reagents used in this assay has been necessary. Inactivation of the AK gene in the expression host used to produce the luciferase in the first place is not an option as AK is an essential enzyme which allows the cell to function. Without this enzyme, the host cell would die.

The applicants have devised a new technique where the problem of contamination of products of recombinant DNA technology by unddesired or even harmful products can be minimised.

In particular, the invention provides a method for producing a polypeptide product which is substantially free of undesired AK, the process comprising culturing a host cell which is able to express said polypeptide product and which is able to express said undesired AK only in a mutant form which form has the activity of the corresponding native AK under culture conditions but is unstable under temperature conditions at which the said polypeptide product remains stable; and recovering the desired product, wherein either the host cell culture or the recovered product is subjected for a sufficient period of time to the temperature conditions under which the undesired AK is unstable so as to denature the undesired AK.

There are several ways which would be clear to a person skilled in the art of how the host chromosomal gene can be inactivated, which is a prerequisite to expressing the undesired product in mutant form only. An example is the use of suicide vectors where the chromosomal gene becomes deactivated and the host is reliant on a plasmid gene.

In this manner, the undesired AK, which is an essential protein for the host cell, is produced during the culture process so that the host cell can continue to multiply. Once the production is complete, a suitable batch of the culture medium is subjected to temperature conditions under which the undesired protein is denatured. Further multiplication of the host cells is not required at this point and so the death of the cells is immaterial. The desired product may then be recovered from the culture residue.

Alternatively, the desired product may be recovered from the culture medium and then subjected to the temperature conditions under which the undesired AK is denatured, so that any contaminant protein will not adversely affect the product activity.

As used herein the term "polypeptide product" refers to any polypeptide which may be expressed from a host cell, including large polypeptides and proteins.

The sorts of conditions which may be used to bring about a denaturation of the undesired AK are conditions of temperature. The mutant AK is made to be thermolabile at temperatures at which the desired product remains intact.

Suitable mutant forms of the protein may be identified by known techniques. For example, random mutagenesis can be used to produce a range of mutant genes. These may be cloned into expression hosts such as *E. coli* using conventional technology and the resultant clones screened for the desired instability. Once identified, the clones which are thermolabile are sequenced and the mutation which results in this property are identified. More than one mutation may result in the labile nature required, and it may be preferable to form mutants with two or more such mutations as this may increase the observed effect. Various techniques are known to the skilled person of providing the undesirable AK in thermolabile form .For example any α-helix portions of the can be made bulkier and thus less thermostable by substituting particular amino acid residue at particular locations. An example of how a labile variant of adenylate kinase can be produced is described in the paper, "Substitution of a serine residue for proline-87 reduces catalytic activity and increases susceptibility to proteolysis of *E*. *coli* adenylate kinase." by A Gilles *et al* in Natl. Acad. Sci. USA, Vol 83, pp 5798-5802, August 1986.

For example, the gene which encodes adenylate kinase of *E. coli* is known (Brune, Schumann and Witinghofer, Nucleic Acids. Research,(1985) 13, No. 19, 7139-7150; P. Liang et al., Gene (1989) 80, 21-28).

In a particular embodiment of the invention, colonies of *E*. *coli* are mutated by non-specific methods and the mutants differentially screened at 25°C and 37°C. Mutants containing a thermolabile adenylate kinase gene should only be able to grow at 25°C and not at 37°C. These can then be screened for AK activity at various temperatures to select out AK mutants.

It has been found that mutation at position 87 in that sequence and/or position 107 in the sequence, produces a mutant form of adenylate kinase enzyme which is labile at low temperatures.
Therefore, an alternative approach is to clone the adenylate kinase gene into a suitable vector such as Promega plasmid "pALTER-1". Site-directed mutagenesis of the amino acids at positions 87 and 107 for example using PCR based methods will give a gene product which has alterered thermolability. Screening of these mutants as described above will indicate which substituent amino acids at these positions give adenylate kinase which is more thermolabile that the native protein.

The construct thus obtained can then be used to transform a competent host strain of *E*. *coli* that is amenable to recombination such as JM83, in order to allow recombination of the mutant gene into the host chromosome, preferably in place of the existing adenylate kinase gene. Successful recombinants will only contain the mutant adenylate kinase gene encoding a thermolabile product. Adenylate kinase is an essential enzyme to the cell; therefore mutants will only be able to survive at a lower permissive temperature than wild type or standard laboratory strains.

Conversely and additionally, the desired polypeptide product may be engineered so that its tolerance to the temperature conditions under which the undesired AK is denatured is increased. For instance, in the case of luciferase enzyme, several thermostable mutants are known in the art and these may be employed in the method of the invention. Alternatively other thermostable mutants can be prepared using similar techniques. in this case, the screening process will select those mutants which have increased tolerance rather than decreased tolerance to the temperature condition being used to denature the undesired polypeptide.

Constructs of the type described above as well as host cells transformed with said constructs and methods of producing them form further aspects of the invention.

Thus the invention further provides a recombinant cell which comprises a first nucleotide sequence which encodes a desired polypeptide such as a luciferase, under the control of regulatory elements which allow expression of said polypeptide, and wherein a gene which encodes AK, which is undesirable as a contaminant in preparations of said polypeptide, is mutated such that the AK, expressed is unstable under temperature conditions in which the polypeptide product remains stable.

Preferably the luciferase is a thermostable luciferase, whilst the adenylate kinase is a thermolabile mutant.

These recombinant cells may contain one or more selection markers which are used in the production process.

The recombinant cell is a prokaryotic cell such as a recombinant *E. coli* cell

The invention further provides a method for producing a recombinant cell according to any one of claims 6 to 8 which method comprises in any order (a) transforming a host cell with a vector which encodes said undesired AK in a form which is unstable under given temperature conditions, subjecting transformants to said temperature conditions and detecting those in which AK is denatured, and (b) transforming said host cell with a vector which encodes a desired polypeptide which is stable under said temperature conditions and a first selection marker, and using the first selection marker to detect stable transformants.

The vector which encodes said undesired AK in a form which is unstable under given temperature conditions may further comprise a selection marker which is different to said first selection marker, and stable transformants are selected.

Suitable selection markers comprise particular different antibiotic resistance genes.

The invention will now be particularly described by way of example.

### Example 1

PCR primers can be designed as is conventional in the art with a view to amplifying a gene encoding adenylate kinase. In this case, AK1 and AKR1013 from Cruachem may be used either as provided or optimised for instance using the Perkin Elmer 2400, and then using the Sigma PCR optimisation kit.

The PCR product which will be the wild-type adenylate kinase gene or alternatively a mutant adenylate kinase gene already known to produce thermolabile adenylate kinase such as strain CV2 is then cloned into a suitable vector, such as the pALTER-1 from Promega, a plasmid based on pBR322. This has disabled antibiotic resistance genes to facilitate mutagenesis. [CV2 is known
(Proc. Natl. Acad. Sci. USA, (1970) 65:737) and may be obtained from the *E*. *coli* Genetic Stock Centre, 355 Osborn Memorial Laboratories, Box 208104, Yale University, New Haven, CT06520-8104, USA.]

In one embodiment, two adenylate kinase genes can be inserted in tandem separated by an antibiotic resistance gene. The elements of the construct may each require individual promoter sequences, but increased expression would result.

Mutations can then be introduced, for example at amino acid positions 87 where proiine may be changed to serine for instance (which involves only one base change) and/or at amino acid position 107 where leucine may be changed for instance to glutamine. The preferred codon usage for adenylate kinase in *E coli* is known. Thus all possible amino acids can be substituted at these positions, and the most thermolabile enzyme which still retains enough activity to allow cell survival will be selected. Preferably, the construct further contains a selection marker gene such as an antibiotic resistance marker downstream of the adenylate kinase gene so that screening for positive recombinants may be effected easily. The selection marker will have to be different to the one used later in the procedure in order to select positive transformants as described below.

Once a suitable thermolabile adenylate kinase producing mutant has been found, the mutated plasmid can be used to transform a recombinant positive strain of *E*. *coli* such as JM83 as is known in the art. The recombinants will then be screened for example by differential screening at 37°C and a much lower temperature, for example 20°C. Positive recombinants may be identified using the selection marker, for example antibiotic resistance where such a marker is present.

Colony blots can then be performed with the resultant mutant oligo to confirm that the mutant sequences are the desired ones. This will be done using chemiluminescent (e.g., HRP) labelled probes. Sequencing can also be carried out in addition to or as well as colony blots.

The *E*. *coli* host produced in this way has a chromosomal mutation in its adenylate kinase gene that causes the adenylate kinase produced to be temperature sensitive. A luciferase plasmid, preferably a thermostable luciferase plasmid, for example as desribed in European Patent Application No. 92110808.0 or WO95/25798, can then be introduced to the host such that the luciferase can be produced at a temperature that is permissive to the adenylate kinase. The culture can be raised to a higher temperature to denature the adenylate kinase which is present.

Purification of the thermostable luciferase enzyme can then be carried out using the standard methods devised previously for example as disclosed in WO95/25798.

## Claims

1. A method for producing a desired polypeptide product which is substantially free of adenylate kinase, the method comprising culturing a prokaryotic cell which is able to express said polypeptide product and which is able to express adenylate kinase, only in a mutant form, which form has the activity of the corresponding native adenylate kinase under culture conditions but is unstable at temperatures at which the said polypeptide product remains stable; and recovering the said polypeptide product, wherein either the prokaryotic cell culture, after culture, or the recovered polypeptide product is subjected, for a sufficient period of time, to temperatures under which the adenylate kinase is unstable so as to denature it.

2. A method according to claim 1 wherein the prokaryotic cells are cultured for a period which is sufficient to allow production of polypeptide product, and then a batch of the culture is subjected to temperatures under which the adenylate kinase is unstable for a sufficient period of time to denature the adenylate kinase, and the polypeptide product is recovered.

3. A method according to claim 1 or 2 wherein the adenylate kinase is thermolabile at a temperature of 37°C or more and the polypeptide product, after culture, is subjected for a sufficient period of time, to temperatures of 37°C or more in order to denature the adenylate kinase.

4. A method according to any one of the preceding claims wherein the polypeptide product is luciferase.

5. A method according to any preceding claim wherein the adenylate kinase includes mutations at amino acids 87 or 107 in the sequence of the *E. coli* adenylate kinase.

6. A recombinant prokaryotic cell which comprises a first nucleotide sequence which encodes a desired polypeptide product under the control of regulatory elements which allow expression of said polypeptide product, and wherein a gene which encodes adenylate kinase which is essential for the host cell but which is undesirable as a contaminant in preparations of said polypeptide product is present only in mutated form such that the adenylate kinase expressed is unstable under temperature conditions in which the polypeptide product remains stable.

7. A recombinant prokaryotic cell according to claim 6, which further comprises at least one selection marker.

8. A recombinant prokaryotic cell according to claim 6 or 7 which is a recombinant *E. coli* cell

9. A method for producing a recombinant prokaryotic cell according to any one of claims 6 to 8 which method comprises in any order (a) transforming a prokaryotic host cell with a vector which encodes adenylate kinase in a form which is unstable under given temperature conditions, subjecting transformants to said temperature conditions and detecting those in which adenylate kinase is denatured, and (b) transforming said prokaryotic host cell with a vector which encodes a desired polypeptide which is stable under said temperature conditions and a first selection marker, and using the first selection marker to detect stable transformants.

10. A method according to claim 9 wherein the vector which encodes said adenylate kinase in a form which is unstable under given temperature conditions further comprises a selection marker which is different to said first selection marker, and stable transformants are selected.

11. A method according to claim 9 or claim 10 wherein said selection markers comprise particular different antibiotic resistance genes.

## Patentansprüche

1. Verfahren zur Herstellung eines gewünschten Polypeptids, das im Wesentlichen frei von Adenylatkinase ist, wobei das Verfahren umfasst:
Kultivieren einer prokaryotischen Zelle, die befähigt ist, das Polypeptidprodukt zu exprimieren, und befähigt ist, Adenylatkinase lediglich in Form einer Mutante zu exprimieren, welche die Aktivität der entsprechenden nativen Adenylatkinase unter Kulturbedingungen besitzt, jedoch bei Temperaturen, bei denen das Polypeptid stabil bleibt, nicht stabil ist, und Gewinnen des Polypeptidprodukts, wobei entweder die prokaryotische Zellkultur nach dem Kultivieren oder das gewonnene Polypeptidprodukt während einer ausreichenden Zeitdauer Temperaturen ausgesetzt wird, bei denen die Adenylatkinase nicht stabil ist, um sie so zu denaturieren.

2. Verfahren nach Anspruch 1, bei dem die prokaryotischen Zellen während einer Zeitdauer kultiviert werden, die für die Erzeugung des Polypeptidprodukts ausreichend ist, dann ein Ansatz der Kultur während einer ausreichenden Zeitdauer Temperaturen ausgesetzt wird, bei denen die Adenylatkinase nicht stabil ist, um die Adenylatkinase zu denaturieren, und dann das Polypeptidprodukt gewonnen wird.

3. Verfahren nach Anspruch 1 oder 2, bei dem die Adenylatkinase bei einer Temperatur von 37 °C oder darüber thermolabil ist und das Polypeptidprodukt nach dem Kultivieren während einer ausreichenden Zeitdauer Temperaturen von 37 °C oder darüber ausgesetzt wird, um die Adenylatkinase zu denaturieren.

4. Verfahren nach einem der vorhergehenden Ansprüche, bei dem das Polypeptidprodukt Luciferase ist.

5. Verfahren nach einem der vorhergehenden Ansprüche, bei dem die Adenylatkinase Mutationen bei den Aminosäuren 87 oder 107 in der Sequenz der Adenylatkinase von *E*. *coli* aufweist.

6. Rekombinante prokaryotische Zelle, die eine erste Nucleotidsequenz aufweist, die ein gewünschtes Polypeptidprodukt unter der Kontrolle von regulatorischen Elementen codiert, welche die Expression des Polypeptidprodukts erlauben, und bei der ein Gen, das Adenylatkinase codiert, die für die Wirtzelle essentiell, aber als Verunreinigung in Präparationen des Polypeptidprodukts unerwünscht ist, lediglich in einer solchen mutierten Form vorliegt, dass die exprimierte Adenylatkinase unter Temperaturbedingungen, unter denen das Polypeptidprodukt stabil bleibt, nicht stabil ist.

7. Rekombinante prokaryotische Zelle nach Anspruch 6, die ferner mindestens einen Selektionsmarker enthält.

8. Rekombinante prokaryotische Zelle nach Anspruch 6, die eine rekombinante Zelle von *E*. *coli* ist.

9. Verfahren zur Herstellung einer rekombinanten prokaryotischen Zelle nach einem der Ansprüche 6 bis 8, das folgende Schritte in beliebiger Reihenfolge umfasst:
(a) Transformieren einer prokaryotischen Wirtzelle mit einem Vektor, der Adenylatkinase in einer Form codiert, die unter gegebenen Temperaturbedingungen nicht stabil ist, Einwirkenlassen dieser Temperaturbedingungen auf die Transformanten und Ermitteln der Transformanten, bei denen Adenylatkinase denaturiert ist, und
(b) Transformieren der prokaryotischen Wirtzelle mit einem Vektor, der ein gewünschtes Polypeptid, das unter diesen Temperaturbedingungen stabil ist, und einen ersten Selektionsmarker codiert, und Verwenden des ersten Selektionsmarkers zur Ermittlung stabiler Transformanten.

10. Verfahren nach Anspruch 9, bei dem der Vektor, der die Adenylatkinase in einer Form codiert, die unter gegebenen Temperaturbedingungen nicht stabil ist, ferner einen Selektionsmarker enthält, der vom ersten Selektionsmarker verschieden ist, und stabile Transformanten selektiert werden.

11. Verfahren nach Anspruch 9 oder 10, bei dem die Selektionsmarker spezielle, unterschiedliche Antibiotikumresistenzgene umfassen.

## Revendications

1. Procédé de production d'un produit polypeptidique souhaité qui est sensiblement dénué d'adénylate kinase, le procédé comprenant la culture d'une cellule procaryote, capable d'exprimer ledit produit polypeptidique et capable d'exprimer l'adénylate kinase, uniquement sous une forme mutante, ladite forme a l'activité de l'adénylate kinase native correspondante dans des conditions de culture mais est instable à des températures auxquelles ledit produit polypeptidique reste stable ; et la récupération dudit produit polypeptidique, dans lequel la culture de cellule procaryote, après culture, ou le produit polypeptidique récupéré est soumis, pendant un laps de temps suffisant, à des températures auxquelles l'adénylate kinase est instable, de façon à la dénaturer.

2. Procédé selon la revendication 1, dans lequel les cellules procaryotes sont cultivées, pendant un laps de temps suffisant, pour permettre la production du produit polypeptidique, et ensuite un lot de la culture est soumis à des températures auxquelles l'adénylate kinase est instable, pendant un laps de temps suffisant pour dénaturer l'adénylate kinase et le produit polypeptidique est récupéré.

3. Procédé selon la revendication 1 ou 2 dans lequel l'adénylate kinase est thermolabile à une température de 37°C ou plus et le produit polypeptidique, après culture, est soumis, pendant un laps de temps suffisant, à des températures de 37°C ou plus, afin de dénaturer l'adénylate kinase.

4. Procédé, selon l'une quelconque des revendications précédentes, dans lequel le produit polypeptidique est la luciférase.

5. Procédé, selon l'une quelconque des revendications précédentes, dans lequel l'adénylate kinase comprend des mutations aux acides aminés 87 ou 107 dans la séquence de l'adénylate kinase *d'E.coli.*

6. Cellule procaryote recombinante qui comprend une première séquence de nucléotide codant un produit polypeptidique souhaité, sous le contrôle d'éléments régulateurs qui permettent l'expression dudit produit polypeptidique, et dans laquelle un gène qui code l'adénylate kinase, qui est essentielle pour la cellule hôte, mais qui est indésirable en tant que contaminant dans les préparations dudit produit polypeptidique, est présent uniquement sous forme mutée, de sorte que l'adénylate kinase exprimée est instable dans des conditions de température auxquelles le produit polypeptidique reste stable.

7. Cellule procaryote recombinante, selon la revendication 6, qui comprend en outre au moins un marqueur de sélection.

8. Cellule procaryote recombinante, selon la revendication 6 ou 7, qui est une cellule *d'E.coli* recombinante.

9. Procédé de production d'une cellule procaryote recombinante, selon l'une quelconque des revendications 6 à 8, ledit procédé comprenant dans n'importe quel ordre (a) la transformation d'une cellule hôte procaryote avec un vecteur qui code l'adénylate kinase sous une forme qui est instable dans des conditions de température données, en soumettant les transformants auxdites conditions de température et en détectant ceux dans lesquels l'adénylate kinase est dénaturée, et (b) la transformation de ladite cellule hôte procaryote avec un vecteur qui code un polypeptide souhaité, qui est stable dans lesdites conditions de température et un premier marqueur de sélection, et en utilisant le premier marqueur de sélection pour détecter des transformants stables.

10. Procédé, selon la revendication 9, dans lequel le vecteur qui code ladite adénylate kinase, sous une forme qui est instable dans des conditions de température données, comprend en outre un marqueur de sélection qui est différent dudit premier marqueur de sélection, et des transformants stables sont sélectionnés.

11. Procédé, selon la revendication 9 ou 10, dans lequel lesdits marqueurs de sélection comprennent des gènes de résistance aux antibiotiques différents particuliers.
